Europäisches Patentamt

(19) European Patent Office

Office européen des brevets

(11) Publication number: **0 341 023**
**A2**

## (12) EUROPEAN PATENT APPLICATION

(21) Application number: **89304403.2**

(22) Date of filing: **02.05.89**

(51) Int. Cl.⁴: **B 01 J 29/02**
C 10 G 11/04, C 10 G 35/06,
C 07 C 5/23

(30) Priority: **02.05.88 US 189460**

(43) Date of publication of application:
**08.11.89 Bulletin 89/45**

(84) Designated Contracting States: **DE FR GB NL**

(71) Applicant: **Exxon Research and Engineering Company**
**P.O.Box 390 180 Park Avenue**
**Florham Park New Jersey 07932 (US)**

(72) Inventor: **Brody, John Francis**
**762 Mountain Avenue**
**Bound Brook New Jersey 08805 (US)**

**Johnson, Jack Wayne**
**12 Sunrise Circle**
**Clinton New Jersey 08809 (US)**

**McVicker, Gary Brice**
**P. O. Box 427, Main Street**
**Califon New Jersey 07830 (US)**

(74) Representative: **Somers, Harold Arnold et al**
**ESSO Engineering (Europe) Ltd. Patents & Licences**
**Apex Tower High Street**
**New Malden Surrey KT3 4DJ (GB)**

(54) Method of catalytic cracking, isomerizing or reforming hydrocarbons.

(57) A process for catalytic cracking, isomerizing of reforming of a hydrocarbon feed stock comprises the step of passing said hydrocarbon feed stock through a catalyst bed at cracking, isomerizing or reforming conditions to crack, isomerize or reform said hydrocarbon feed stock, wherein the catalyst composition in the catalyst bed, comprises a pillared fluorotetrasilicic mica, wherein said pillared fluorotetrasilicic mica has a specific surface area exceeding 75 m²/gm, wherein the mica is pillared by alumina, zirconia or chromia, and the aluminum, zirconium or chromium content is at least 8% by weight. In the catalytic cracking process, there is less carbon and hydrogen formed than in comparative processes, and the cracked product contains a relatively high proportion of $C_3$ and $C_4$ olefins.

EP 0 341 023 A2

Description

## METHOD OF CATALYTIC CRACKING, ISOMERIZING OR REFORMING HYDROCARBONS

SUMMARY OF THE INVENTION

This invention is a pillared synthetic fluorotetrasilicic mica which can be used as a fixed bed or in a fluidized catalytic cracking unit wherein the pillared fluorotetrasilicic mica is an active ingredient in the catalytic composition being used in the fluidized catalytic cracking unit in a hydrocarbon cracking process or a hydrocarbon isomerization process. It has an included aluminum content of at least 7%; a BET surface area of more than 75 m$^2$/gm, preferably between 200 m$^2$/gm to 400 m$^2$/gm; and an average interlayer spacing as measured by x-ray diffraction of between 17 Å and 19 Å. The x-ray diffraction pattern may be as follows:

| d Å (hkl) | 2θ (CuKα) | I/Io |
|---|---|---|
| 18.7 (001) | 4.7 | 100 |
| 9.5 (002) | 9.2 | 8 |
| 4.53 (020,110,004) | 19.6 | 25 |
| 3.72 (005) | 23.9 | 5 |
| 3.14 (006) | 28.4 | 15 |
| 2.56 (130, 200) | 35.0 | 27 |
| 1.72 (150) | 53.3 | 4 |
| 1.52 (060) | 61.0 | 15 |

The first peak of the diffraction pattern is always observed. The other peaks may or may not be observed, depending on minor variations in the preparation conditions. These materials are thermally and hydrothermally stable at relatively high temperatures and consequently may be used as supports for catalysts or, in some instances, as catalysts per se.

The process used to produce this material begins with a water-swelling fluorotetrasilicic mica, preferably of the formula Na(Mg$_{2.5}$Si$_4$O$_{10}$F$_2$). The Na TSM is preferably size fractionated by, e.g., dispersal in a suitable solvent and centrifugation. The preferred size is less than 0.2 microns although larger size fractions, e.g., 0.2 to 2.0 microns, are also useful. An aluminum polyoxocation solution is then added to an aqueous slurry of the Na TSM in an amount larger than about 3.0 meq Al$_{13}^{7+}$ per gram of mica. The preferred aluminum polyoxycation is Al$_{13}$O$_3$(OH)$_{24}$(H$_2$O)$_{12}^{7+}$ or similar polyoxocations. The pH in the aluminum polyoxocation-Na TSM slurry must be in the acid region and sufficient aluminum in the slurry must be present or the resulting product is either partially collapsed upon heating or does not form a pillared product. The slurry is, if desired, heated to less than 200°C for a period of up to half a day. The resulting products are filtered, washed, and dried. The product, after drying, may be calcined by a staged process for several hours. After calcination, some residual alkali metal is labilized and may be removed by washing the pillared mica product with water or an aqueous ammonium salt solution. The washed product has enhanced thermal stability.

This invention relates to an improved hydrocarbon cracking process of a liquid hydrocarbon feed stock for substantially reducing the formation of hydrogen and carbon in the process, wherein the process comprises the step of passing said hydrocarbon feed stock through a catalytic cracking bed at a temperature of 400°C to 600°C for a sufficient period of time to convert said liquid hydrocarbon feed stock into a product containing a high percentage of C$_3$ and C$_4$ olefins, wherein the improvement comprises said catalytic cracking bed being a pillared fluorotetrasilicic mica in the catalytic composition wherein said pillared fluorotetrasilicic mica has a specific surface area of greater than 75 m$^2$/gm, wherein the mica is pillared by alumina or zirconia and the aluminum or zirconium content is at least 8% by weight.

BRIEF DESCRIPTION OF THE DRAWINGS

Figure 1 illustrates X-ray powder diffraction patterns (CuKα radiation) of pillared TSM as a function of heat treatment, see Example 4.

Figure 2 illustrates X-ray powder diffraction patterns of pillared TSM as a function of thermal treatment, see Example 5.

Figure 3 illustrates X-ray powder diffraction patterns (CuKα radiation) and surface areas of pillared TSM as a function of thermal treatment, see Example 6.

Figure 4 illustrates X-ray powder diffraction patterns (CuKα radiation) and surface areas of washed, pillared TSM as a function of steam treatment, see Example 7.

Figure 5 illustrates X-ray powder diffraction patterns (CuKα radiation) of washed, pillared taeniolite as a function of steam treatment, see Example 10.

Figure 6 illustrates the X-ray powder diffraction diagram of Zr-pillared TSM (CuKα radiation).

Figure 7 illustrates the hexane absorption isotherm on Zr-pillared TSM.

EP 0 341 023 A2

## DESCRIPTION OF THE PREFERRED EMBODIMENTS

This invention relates to an improved hydrocarbon cracking process of a liquid hydrocarbon feed stock for substantially reducing the formation of hydrogen and carbon in the process wherein the process comprises the step of passing said hydrocarbon feed stock through a catalytic cracking bed at a temperature of 450°C to 500°C for a sufficient period of time to convert said liquid hydrocarbon feed stock into a product containing a high percentage of $C_3$ and $C_4$ olefins, wherein the improvement comprises said catalytic cracking bed being a pillared fluorotetrasilicic mica in the catalytic composition wherein said pillared fluorotetrasilicic mica has a specific surface area of greater than 75 $m^2$/gm, wherein the mica is pillared by alumina or zirconia and the aluminum or zirconium content is at least 8% by weight.

The invention also relates to an improved hydrocarbon isomerization process of an olefin for substantially reducing the formation of coke in the process wherein the process comprises the step of pass said olefin through a fixed catalytic bed at a temperature of 250°C to 400°C for a sufficient period of time to isomerize said olefin into a product containing a minimum amount of coke, wherein the improvement comprises a fixed bed being a pillared fluorotetrasilicic mica in the catalytic composition, wherein said pillared fluorotetrasilicic mica has a specific surface area of greater than 75 $m^2$/gm, wherein the mica is pillared by alumina or zirconica and the aluminum or zirconium content is at least 8% by weight.

This invention also relates to an improved hydrocarbon reforming process for the conversion of an hydrocarbon alkane into an aromatic hydrocarbon, wherein the process comprises the step of passing said hydrocarbon alkane through a fixed catalytic bed to convert said hydrocarbon alkane into said aromatic hydrocarbon wherein the improvement comprises a fixed catalytic bed which has as an active ingredient a pillared fluorotetrasilicic mica in the catalytic composition, wherein said pillared fluorotetrasilicic mica has a specific surface area of greater than 75 $m^2$/gm, wherein the mica is pillared by alumina or zirconic and the aluminum or zirconium content is at least 8% by weight. The said pillared mica has from 0.1 to 5.0% by weight of a noble metal such as Pt, Pd, or the like deposited on it.

As mentioned above, the starting materials used to prepare the pillared micas of the instant invention are all water swellable, fluorotetrasilicic micas. The preferred form is NaTSM, but the sodium ion may be partially replaced by other ions such as lithium, lead, strontium or mixtures of all four. Other fluorotetrasilic micas such as lithium taeniolite, Li[LiMg$_2$Si$_4$O$_{10}$F$_2$] may also be employed.

The pillaring agent used in the practice of the present invention may be a salt pair or a neutral molecule but preferably is an aluminum polyoxocationic material. As noted above, the preferred material is an aluminum chlorohydrol solution. This material is believed to contain the cation $Al_{13}O_4(OH)_{24}(H_2O)_{12}^{7+}$ although this is not known with any certainty. Generically the pillaring medium must produce in the interlayer of the mica stable pillars after the heating steps disclosed below.

An especially suitable process for pillaring the tetrasilicic fluoromica includes the steps of first finely dividing and dispersing the fluoromica in water to produce a thin slurry. The step of finely dividing the mica should preferably produce particles in the size range less than 0.2 microns. However, slightly larger particles, e.g., in the range of 0.2 to 2.0 microns, will also operate in this process although in many commercial Na TSM materials several additional phases, notably cristobalite, are present and do not add to the reaction product. The slurry may then be neutralized with a suitable acid, e.g., HCl or HNO$_3$, to a pH of less than 7, preferably in the range 4.5-6, and then allowed to stand so as to settle out non-dispersed solids. The upper layer containing the dispersed mica may then be separated to yield a mica precursor suspension, e.g., about 1% solids. This suspension may be used as the pillaring feed after suitable dilution or the suspension may be concentrated by e.g., freeze drying, to produce a solid which is then redispersed in a polar solvent such as water, glycol, or alcohol. The suspension is preferably produced in an aqueous form. The suspension is then intimately mixed with an appropriate amount of an aqueous solution of the pillaring medium. The mixed suspension is then desirably heated at a low level. The temperature is not particularly critical in that anything between about room temperature and below 200°C is generally acceptable. Obviously the reaction vessel must be capable of withstanding the autogeneous pressure resulting from such a reaction step.

The resulting flocculated solids may be separated from the solution by filtration or centrifugation and are preferably washed thoroughly with distilled water. They may typically be dried at 100°C to 140°C. The reaction products may then be calcined in an oxidizing or neutral atmosphere.

The catalytic and absorbent characteristics of the pillared interlayered micas of the present invention may be modified with a wide range of cations including hydrogen, ammonium and metals of Groups IB through VIII of the Periodic Table. These products are particularly useful for reactions taking place below 700°C and requiring high specific surface area.

In general, the heat treated pillared micas of this invention have an interlayer spacing of between 16 Å and 20 Å; preferably between 17.5 Å and 19 Å. The BET surface area is between 75 $cm^2$/gm and 400 $cm^2$/gm; preferably between 200 $cm^2$/gm and 350 $cm^2$/gm. The X-ray diffraction pattern typically displays one or a series of 001 maxima derived from the layer spacing, along with mica hkO bands at d values of 4.53, 2.56 and 1.52 Å.

The heat-treated materials have an aluminum content in the range of 8 to 14% Al by weight. Of course, if an impure fraction of fluorotetrasilic mica is used in the pillaring procedure which contains non-swelling phases (e.g., cristobalite) which do not accept the aluminum polycations, the amount of aluminum in the resulting product will be proportionately lower.

3

The heat-treated materials have a residual interlayer alkali content that is not removed by repeated washing before the calcination step. The calcination process labilizes the residual alkali, and by washing the heat treated material with water or an aqueous ammonium salt solution, a significant fraction of the residual alkali is removed. The resulting washed, pillared mica shows enhanced thermal stability, as demonstrated in the examples.

As well as the above-mentioned pillared fluorotetrasilicic micas which have pillars comprised of alumina, other metallic oxides can be used to form the pillars in the pillared micas of this invention. For example, chlorides and oxychlorides of zirconium and chromium, when hydrolyzed by water and/or base can form hydroxyaquo oligimeric cations which can serve as the pillar precursors in the pillared fluorotetrasilicic micas.

These pillared micas are useful as absorbents and catalytic supports. They may be combined with other inorganic oxides such as silica, alumina, silica-alumina, natural or synthetic zeolites, and other clays. Because of their surface area and pore volume, they are believed to be particularly useful as catalyst matrix materials for catalytic cracking of heavy hydrocarbons.

Having described the basic and broad aspects of the invention, the following specific examples are given to illustrate preferred embodiments and are not offered to limit, in any way, the scope of the invention.

## EXAMPLE 1

A sample of 40 grams of an Na-TSM synthetic product supplied by TOPY Industries of Japan was dispersed in distilled water (2400 ml) by vigorous mixing in a blender. The pH of the resulting suspension was adjusted to 5 while mixing in the blender and the entire sample was transferred to four 1000 ml cylinders to stand undisturbed for 3 hr. After the sedimentation period, the supernatant was siphoned off, leaving 16 grams coarse sediment that was discarded. The supernatant was centrifuged for 1 hr. at 2400 rpm. The resulting supernatant suspension that remained after centrifugation was decanted into stainless steel trays and freeze-dried, yielding 15.4 g of fluffy white solid. X-ray diffraction showed a layer spacing of 12.4 Å and the absence of the minor impurities cristobalite, amphibole, and $MgF_2$ that were present in the unfractionated sample. The BET surface was 10 $m^2/g$.

The intermediate size fraction was isolated by freeze drying the sediment obtained in the centrifugation step, yielding 4.6 g of solid. X-ray diffraction showed substantial amount of a cristobalite ($SiO_2$) impurity phase in addition to the Na-TSM.

A 1 g portion of the fine fraction was again dispersed in distilled water (300 ml). After the TSM was completely dispersed by stirring the suspension overnight, aluminum chlorohydrol solution (1.20 g, Chlorhydrol 50S, Reheis Chemical) was added. The resulting viscous suspension was heated to 90°C for one hour while stirring. The mixture was allowed to cool and the solid was separated by filtration, washed thoroughly with distilled water, and dried at 120°C. X-ray diffraction showed the layer spacing to be 19.2 Å. The sample was then placed in a muffle furnace at 200°C for 2 hr., heated to 400°C at 50°C/hr., and held at 400°C for 2 hr. The layer spacing after this treatment was 18.7 Å. The aluminum content was 9.3% by weight.

Nitrogen adsorption and desorption studies indicated the calcined sample had a surface area of 394 $m^2/g$ and a pore volume of 0.29 ml/g. The pore volume was calculated from the desorption isotherm and does not include the micropores of a radius less than 10 Å.

## EXAMPLE 2

(Comparative)

The process of Example 1 was repeated but the whole fraction of Na TSM, as received, was mixed with 1.5 meq $Al_{13}^{7+}$ (0.6 g Chlorhydrol 50S/gm mica) in a 1% aqueous suspension. No pH adjustment was made. Only a slight flocculation upon addition of the aluminum oligomer solution was noted. After drying at 120°C, the x-ray diffraction pattern showed spacings of 16.2 and 13.9 Å rather than the single spacing at 18-19 Å typical of a successfully pillared material. The acid balance and higher aluminum concentration in Example 1 clearly result in the production of a pillared material.

## EXAMPLE 3

The pillaring process of Example 1 was repeated using an intermediate size fraction (0.2 -2.0 μ) of the acid balanced Na-TSM. Following calcination at 400°C, a pillared TSM having a 17.8 Å layer spacing and a surface area of 218 $m^2/gm$ was produced. The x-ray diffraction pattern showed the clear presence of cristobalite as a separate phase.

EP 0 341 023 A2

## EXAMPLE 4

The product of Example 1 was heated for 2 hr. in air at successively increasing temperatures and examined by X-ray diffraction to assess its thermal stability. The results are presented in the following table.

| Temperature (°C) | d(001) (Å) |
|---|---|
| 500 | 18.2 |
| 544 | 18.3 |
| 604 | 17.8 |
| 652 | 17.7 |
| 706 | 16.6 |
| 756 | 10.5 |

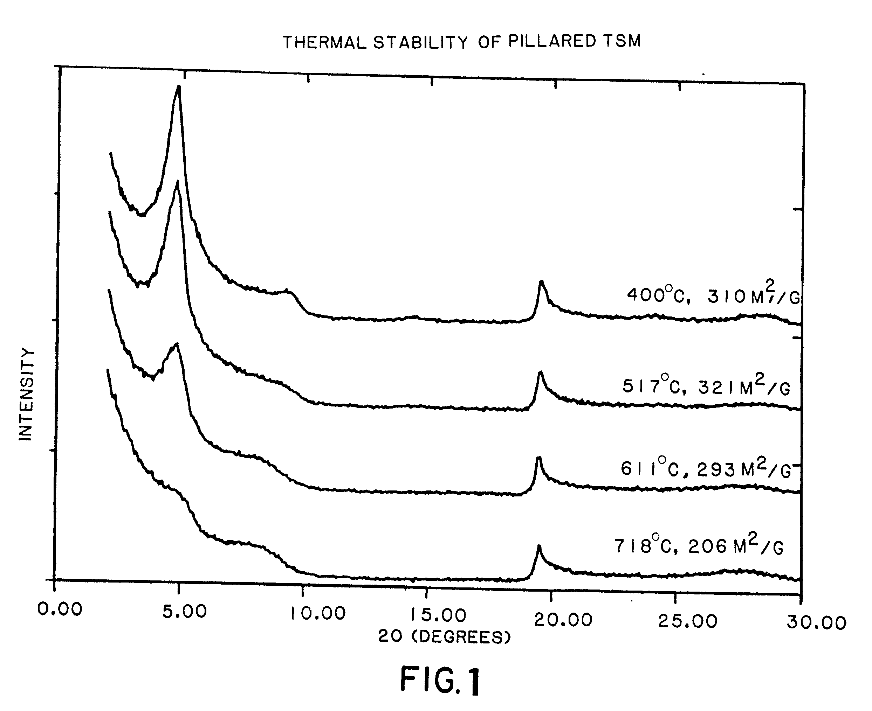

As can be seen in Figure 1, the crystallinity suffers with heat treatment, but the layers remain expanded up to 700°C.

## EXAMPLE 5

In a series of samples prepared separately from those of Example 1, pillared TSM was calcined at successively higher temperatures for two-hour periods and the effect on both the surface area and the layer spacing was measured with the results as follows:

| Temperature (°C) | d(001) (Å) | Surface area (m²/g) |
|---|---|---|
| 400 | 18.7 | 313,306 |
| 517 | 18.5 | 321 |
| 611 | 18.5 | 293 |
| 718 | shoulder | 206 |

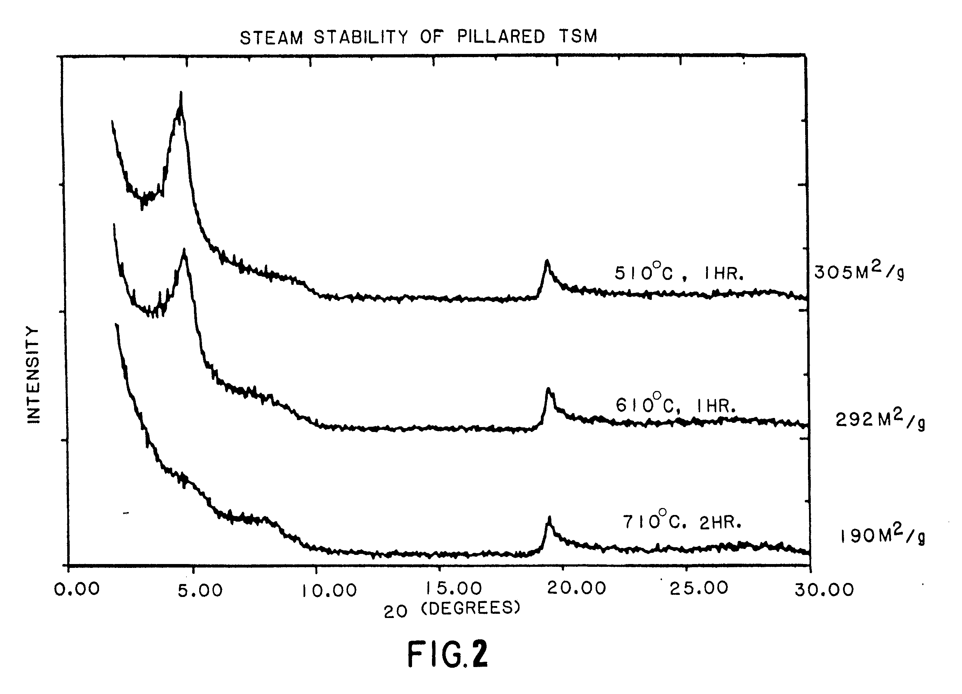

The pillared mica shows excellent thermal stability at 610°C. At 718°C the surface area only decreases by a third, even though the regularity in the layer spacing as determined by X-ray diffraction is almost lost. These X-ray diffraction patterns are shown in Figure 2.

## EXAMPLE 6

Separate samples of calcined pillared TSM prepared as in Example 1 were heated in an atmosphere of steam maintained by passing a stream of helium through a room temperature water reservoir and then through the furnace in which the sample was heated, resulting in a $H_2O$ partial pressure of ca. 18 Torr. The samples were heated in a steam atmosphere for 1 hour periods, then examined by X-ray diffraction and BET surface area analysis with the following results:

| Temperature °C | d(001) (Å) | Surface area (m²/g) |
|---|---|---|
| 511 | 18.4 | 306 |
| 610 | 18.2 | 292 |
| 710 | -- | 190 |

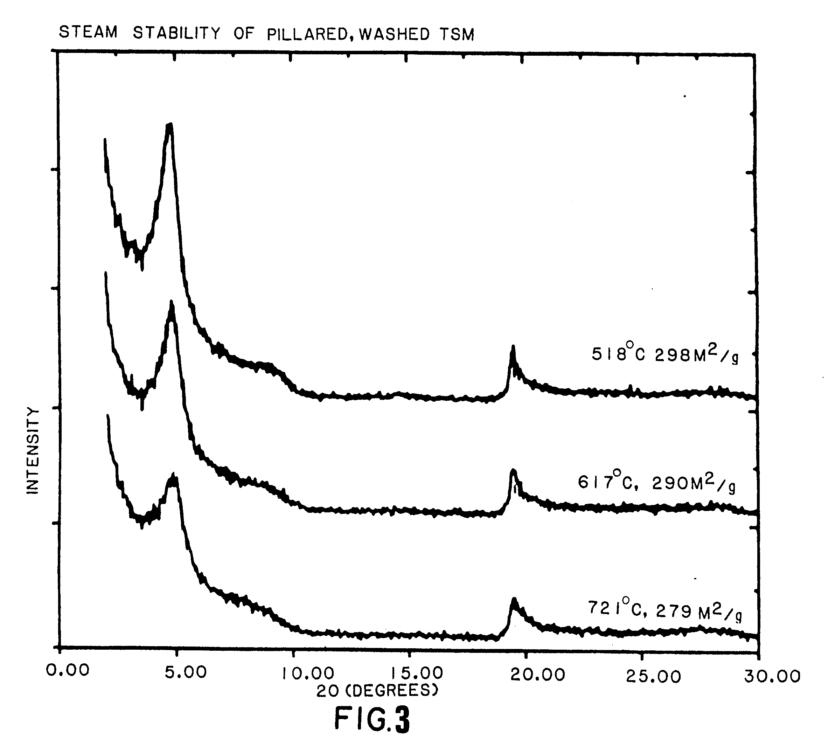

These results are very similar to those of Example 5, showing that the thermal stability is not adversely affected by the presence of steam. They are shown in Figure 3.

## EXAMPLE 7

Where pillared TSM is produced by the method of Example 1, all of the sodium is not removed by the exchange with aluminum polyoxocations. Usually 1.0 wt% Na remains in the pillared mica, even after exhaustive washing. However, after the pillared mica has been calcined at 400°C, the sodium is labilized, and can be partially (40-60%) removed by simple water washing. Calcined pillared TSM (1.5 g), prepared as in Example 1, with a measured sodium content of 0.96% and surface area of 306 m²/g was washed three times with 125 ml portions of distilled water and the solid separated by centrifugation. The resulting washed solid

5

EP 0 341 023 A2

was dried and calcined to 400°C as in Example 1. The resulting washed pillared TSM had an X-ray diffraction pattern indistinguishable from that of the precursor, a surface area of 294 $m^2/g$, and a reduced sodium content of 0.47%. The washed pillared mica was steam treated as in Example 6, with the following results:

| Temperature °C | d(001) (Å) | Surface area $(m^2/g)$ |
|---|---|---|
| 400 | 18.3 | 294 |
| 518 | 18.5 | 298 |
| 617 | 18.1 | 290 |
| 721 | 18.1 | 279 |

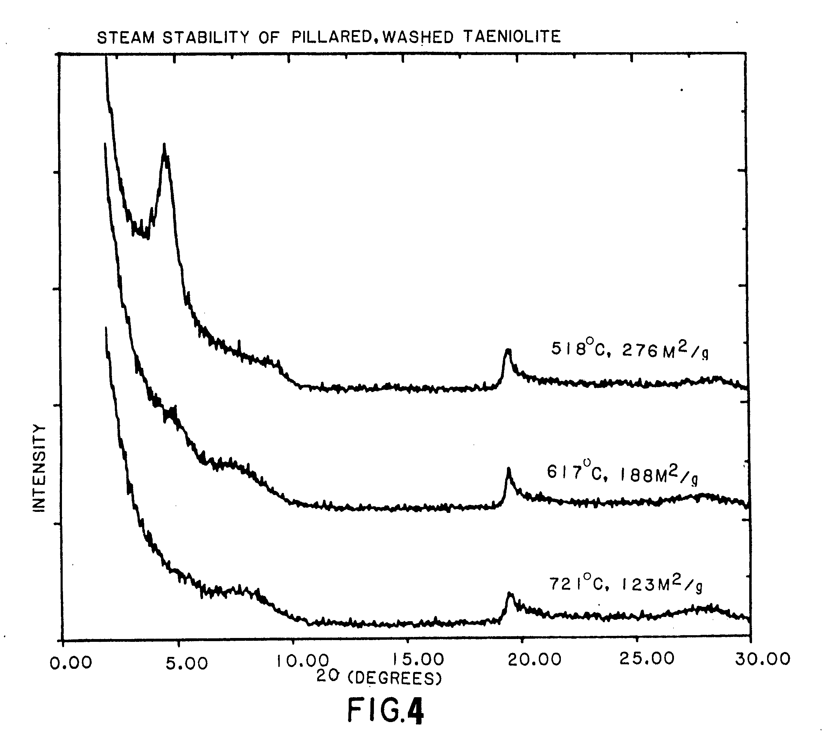

Removal of sodium by washing significantly enhances the stability of the pillared mica. These data are shown in Figure 4.

### EXAMPLE 8

Lithium taeniolite, a synthetic fluorotetrasilic mica with formula $Li[LiMg_2Si_4O_{10}F_2]$ supplied by Topy Industries of Japan was dispersed, acid-balanced, and size-fractionated according to the method of Example 1. 5 g of the fine fraction was dispersed in 3000 ml water by stirring 24 hours. Aluminum polyoxocation solution (6.0 g chlorhydrol 50 S, Reheis Chemical) was added and the solution stirred and heated to 90°C for one hour. After cooling, the solid was separated by centrifugation, washed with five 1000 ml portions of distilled water, and dried at 120°C. X-ray diffraction showed the layer spacing to be 19.0 Å. The solid was calcined in air at 200°C and then heated at 50°C/hr to 400°C and held at 400°C for two hours. The resulting calcined pillared mica had a layer spacing of 19.1 Å, a surface area of 188 $m^2/g$, and an aluminum content of 14.1% by weight.

### EXAMPLE 9

The pillared taeniolite of Example 8 was calcined for successive two hour periods at increasing temperature, then analyzed by X-ray diffraction and BET surface area techniques with the following results:

| Temperature °C | d(001) (Å) | Surface area $(m^2/g)$ |
|---|---|---|
| 508 | 18.4 | 204 |
| 605 | --- | 142 |
| 709 | -- | 65 |

This material is thermally stable to >500°C. Although it is less stable and has lower surface area than pillared TSM, it still may be useful in some applications involving lower temperature service.

### EXAMPLE 10

Calcined pillared taeniolite, prepared as in Example 8, was water washed and recalcined at 400°C following the procedure of Example 7. The surface area after this treatment rose to 264 $m^2/g$ and the layer spacing was 18.6 Å. Steam treatment at successively higher temperatures gave the following results.

| Temperature °C | d(001) (Å) | Surface area $(m^2/g)$ |
|---|---|---|
| 518 | 18.5 | 276 |
| 617 | 17.5 (weak) | 188 |
| 721 | -- | 123 |

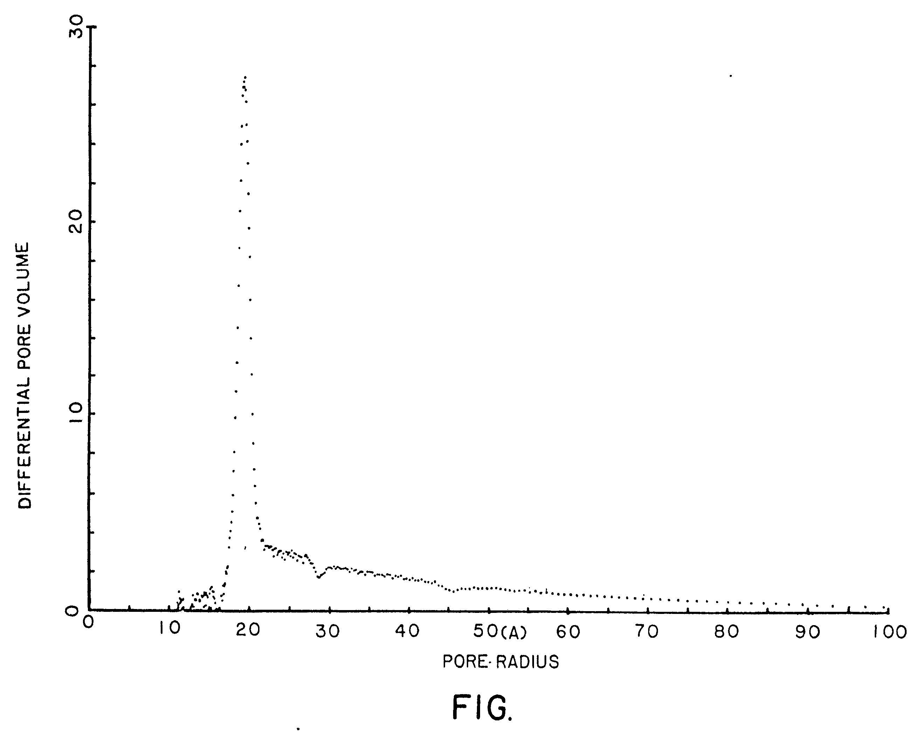

These results are shown in Figure 5.

### EXAMPLE 11

6

### Zirconia-pillared Tetrasilicic Mica

The fine fraction of Na-TSM, isolated as in Example 1, was treated with an aqueous solution of zirconyl chloride, $ZrOCl_2.4H_2O$, in a manner similar to that disclosed by Vaughan, US Patent 4,176,090 for treatment of smectites. $ZrOCl_2.4H_2O$ (1 g) was dissolved in 12 ml $H_2O$ and heated at reflux for 24 hr. The resulting milky white solution was cooled to room temperature and diluted to 100 ml with $H_2O$. 0.65 g of Na-TSM, fine fraction, was added to the $ZrOCl_2$ solution and the mixture was stirred at room temperature for one hour. The product was separated by filtration, washed repeatedly with hot water, and dried at 110°C. The material was then calcined for 2 hr at 200°C, heated at 50°C/hr to 400°C and held at 400°C for 2 hr. Elemental analysis showed the incorporation of 15.1% Zr by weight in the product. The X-ray powder diffraction pattern, shown in Figure 6, exhibits a shoulder at a d spacing of 21 Å. The quality of the pattern shows that the layer spacing in Zr-TSM is not as regular as that in Al-TSM. Zr-TSM does have significant microporosity as shown by the BET surface area of 231 $m^2/g$ and its hexane isotherm, shown in Figure 7. The initial portion of the isotherm shows rapid adsorption of about 5 wt% hexane at low partial pressure due to the micropores. The hysteresis on the desorption branch shows the presence of some mesopores as well.

### EXAMPLE 12

### Chromia-Pillared TSM

Chromia-pillared TSM was prepared in a manner similar to that used by T.J. Pinnavaia, et al., J. Amer. Chem. Soc. 107, 4783 (1985), to make chromia pillared montmorillonite. 5.58g $Na_2CO_3$ was added to a solution of 18 g $Cr(NO_3)_3.9H_2O$ in 450 ml water and the mixture was heated at 95°C for 24 hr. 0.5 g of the intermediate fraction of NaTSM, isolated as in Example 1, was added to the resulting solution of hydroxyaquochromium oligimers and the suspension was held at 95 °C for 1.5 hr. The mixture was cooled, the solid was separated by centrifugation, thoroughly washed with distilled water, and air dried. X-ray powder diffraction showed a sharp maximum corresponding to layer separation of 18.7Å. Elemental analysis indicated the presence of 10.7 wt. % Cr in the pillared product. After calcination at 300°C for 2 hr the 18.8 Å spacing persisted and the BET surface area was 191 $m^2/g$. Further calcination at 400°C resulted in collapse of the mica layers to 10 Å and the appearance of the peaks corresponding to $Cr_2O_3$ in the X-ray powder diffraction pattern. This data high-lights a difference between the micas of this invention and the pillared smectites of the prior art. Chromia-pillared smectites such as montmorillonite and fluorohectorite prepared by this method have higher layer separations, in the range of 26 to 28 Å, than the 18 Å layer separations observed in the chromia-pillared micas of this invention.

### EXAMPLE 13

### Chromia-Pillared Taeniolite

In a manner identical to that of the previous example, the fine fraction of Li taeniolite was pillared with hydroxyaquochromium oligimers. The resulting chromia-pillared taeniolite had a layer separation of 18.9 Å as measured by X-ray powder diffraction. Upon calcination at 300°C for 2 hr the layer separation decreased to 14 Å and the BET surface area was 147 $m^2/g$. Further calcination at 410°C resulted in collapse of the layer spacing to 13 Å and the appearance of $Cr_2O_3$ in the X-ray powder diffraction pattern.

### EXAMPLE 14

### Use of Al-TSM as Cracking Catalyst for Petroleum Fractions

Washed Al-TSM, prepared as in Example 7, was formulated into a fluid cracking catalyst by mixing it with a standard 3A silica-alumina gel and oven drying to form a 20% Al-TSM composite. The resulting material was tested for catalytic activity with a standard MicroActivity Test (MAT), which involves passing a petroleum fraction through a fixed bed of the catalyst at elevated temperature and analysis of the resulting products. Before testing, the catalysts were treated with 100% steam at 600°C for 3 hrs. to simulate an equilibrium catalyst. For comparison, a sample of a standard zeolite catalyst, LZ-Y82, and of an Al-pillared montmorillonite,

7

a standard pillared clay, were treated in an identical fashion. The results for both a light and a heavy feed are presented in Tables 1 and 2. In comparing Al-TSM with Al-montmorillonite, markedly lower yields of both hydrogen and carbon are observed for Al-TSM as compared to the Al-montmorillonite. Both of these are undesirable products in catalytic hydrocarbon cracking, thereby, making the pillared mica an obvious improvement over the prior art. The breakdown of the gas yields (Table 2) shows that both Al-TSM and Al-montmorillonite give substantial enhancements in $C_3$ and $C_4$ olefins when compared to the conventional zeolite catalyst. In spite of lower overall activity, this high olefin selectivity makes the Al-TSM catalysts preferred when higher olefin yields are required.

<u>EXAMPLE 15</u>

<u>Use of Al-TSM as a Hydrocarbon Isomerization Catalyst</u>

Al-TSM prepared as in Example 1 and washed Al-TSM prepared as in Example 7 were tested as hydrocarbon isomerization catalysts by flowing 2-methylpent-2-ene vapor in helium over a fixed bed of the catalyst at 350°C. The rates of conversion to various hydrocarbon products were monitored by gas chromatography. Al-pillared montmorillonite and gamma alumina, with and without added chloride, were also tested under identical conditions for comparison. The rates of conversion to 4-methylpent-2-ene, the product of a 1,3-hydrogen shift, 3 methyl-pent-2-ene, the product of a 1,2-methyl shift, and 2,3-dimethylbut-2-ene, the product of more extensive isomerization, are listed in Table 3. As can be seen from the differences in conversion, the post-calcination water washing significantly enhances the activity of Al-TSM for this acid-catalyzed reaction. Washed Al-TSM is more active than Al-montmorillonite or gamma alumina, and comparable in activity to chlorided gamma alumina. The selectivity results show that the methyl shift is enhanced, resulting in a relatively large amount of 3-methylpent-2-ene. In order to get a comparable methyl shift activity in gamma alumina, it must be chlorided, resulting in the production of very strong acid sites as is shown by the production of 2,3-dimethylbut-2-ene. In isomerization reactions, such strong acid sites often lead to coke formation and are undesirable. This demonstrates that Al-TSM has a relatively large number of acid sites of the proper strength to be a new and useful hydrocarbon isomerization catalyst or catalyst support.

## TABLE 1

### MAT Results on Cracking Catalysts

| | Gas oil (800°F) | | | Cat cycle oil (1000°F) | | |
| | Al-TSM | Al-Mont | LZ-Y8Z | Al-TSM | Al-Mont | LZ-Y8Z |
|---|---|---|---|---|---|---|
| % Conversion | 62.4 | 64.5 | 80.2 | 73.5 | 72.8 | 84.5 |
| % $H_2$ x 100 | 2.03 | 4.82 | 3.70 | 3.05 | 7.81 | 4.92 |
| % Carbon | 2.94 | 3.95 | 5.13 | 4.50 | 6.17 | 6.47 |
| % bottoms (650°F$^+$) | 16.1 | 14.0 | 2.5 | 7.4 | 7.0 | 3.1 |

EP 0 341 023 A2

## TABLE 2

### Gas Yields for MAT on Cracking Catalysts

| | Gas oil (800°F) | | | Cat cycle oil (1000°F) | | |
|---|---|---|---|---|---|---|
| | Al-TSM | Al-Mont | LZ-Y8Z | Al-TSM | Al-Mont | LZ-Y8Z |
| Methane | 0.26 | 0.28 | 0.41 | 0.46 | 0.47 | 0.59 |
| Ethylene | 0.31 | 0.36 | 0.77 | 0.42 | 0.47 | 0.93 |
| Ethane | 0.29 | 0.31 | 0.44 | 0.52 | 0.53 | 1.55 |
| Propylene | 3.21 | 5.47 | 2.78 | 6.64 | 6.70 | 3.15 |
| Propane | 0.53 | 0.63 | 1.67 | 0.77 | 0.85 | 2.13 |
| Butylene | 1.79 | 1.94 | 0.95 | 2.48 | 2.51 | 1.15 |
| Butanes | 2.77 | 3.21 | 5.54 | 3.94 | 4.21 | 6.85 |
| $C_5$ | 1.99 | 1.49 | 2.54 | 2.00 | 2.15 | 3.51 |
| Total | 11.15 | 13.69 | 15.10 | 17.23 | 17.89 | 19.86 |

## TABLE 3

### 2-Methylpent-2-ene Isomerization Results

| | Conversion (mole %) | Selected Rates (moles/hr/g x $10^3$) | | |
|---|---|---|---|---|
| | | 4MP2 | 3MP2 | 23DMB |
| Al-TSM | 37.8 | 1.29 | 0.63 | 0.04 |
| Al-TSM, washed | 62.1 | 3.55 | 3.90 | 0.41 |
| Al-Mont | 57.3 | 3.30 | 2.86 | 0.39 |
| Gamma $Al_2O_3$ | 50.2 | 2.88 | 1.65 | 0.83 |
| | 56.7 | 3.50 | 2.05 | 1.00 |
| Gamma $Al_2O_3$, | 63.1 | 2.63 | 3.54 | 1.47 |
| 0.9% Cl | 62.1 | 3.00 | 2.77 | 1.51 |

## EXAMPLE 16

### Use of Al-TSM as a Hydrocarbon Aromatization Catalyst

Pillared TSM is useful as a reforming catalyst for hydrocarbon fractions when used in combination with a noble metal such as palladium or platinum. To demonstrate the potential of this new catalyst, the conversion of hexane to benzene was chosen as a model reaction. Al-TSM prepared as in Example 1 and washed Al-TSM prepared as in Example 7 were loaded with 1% Pt by weight, reduced at 500°C in hydrogen, and tested as hydrocarbon aromatization catalysts by flowing hexane vapor in hydrogen over a fixed bed of the catalyst at 350°C. The rates of conversion to various hydrocarbon products were monitored by gas chromatography. After 80 minutes reaction at a temperature of 400°C, 63% of the hexane was converted from $C_6$-alkane with a 36% selectivity to benzene using the Al-TSM of Example 1. Under similar conditions using the washed Al-TSM of Example 7, 78% of the hexane was converted from alkane with a 29% selectivity to benzene. This aromatization activity demonstrates the utility of these new catalysts in hydrocarbon reforming applications.

Having thus described the invention by disclosure and by examples, it should be apparent to one having ordinary skill in the art that there are various feed materials and methods of producing the inventive material which would be equivalent to those discussed here but within the spirit of the invention claimed below.

**Claims**

1. A hydrocarbon cracking process of a liquid hydrocarbon feed stock for substantially reducing the formation of hydrogen and carbon in said process which comprises the step of passing said hydrocarbon feed stock through a catalytic cracking bed at a temperature in the range of from 400°C to 600°C for a sufficient period of time to convert said liquid hydrocarbon feed stock into a product containing a high

percentage of $C_3$ and $C_4$ olefins, wherein the catalyst of the said catalytic cracking bed comprises a pillared fluorotetrasilicic mica, wherein said pillared fluorotetrasilicic mica has a specific surface area of greater than 75 m²/gm, wherein the mica is pillared by alumina or zirconia or chromia and the aluminum or zirconium or chromium content is at least 8% by weight.

2. A hydrocarbon isomerization process of an olefin for substantially reducing the formation of coke in said process which comprises the step of passing said olefin through a fixed catalytic bed at a temperature in the range of from 250°C to 400°C for a sufficient period of time to isomerize said olefin into a product containing a minimum amount of coke, wherein the catalyst of the said fixed bed comprises a pillared fluorotetrasilicic mica, wherein said pillared fluorotetrasilicic mica has a specific surface area of greater than 75 m²/gm, wherein the mica is pillared by alumina or zirconia or chromia and the aluminum or zirconium or chromium content is at least 8% by weight.

3. A hydrocarbon reforming process for the conversion of a hydrocarbon alkane into an aromatic hydrocarbon which comprises the step of passing said hydrocarbon alkane through a fixed catalytic bed to convert said hydrocarbon alkane into said aromatic hydrocarbon wherein the catalyst of the fixed catalytic bed has as an active ingredient a pillared fluorotetrasilicic mica, wherein said pillared fluorotetrasilicic mica has a specific surface area of greater than 75 m²/gm, wherein the mica is pillared by alumina or zirconia or chromia and the aluminum or zirconium or chromium content is at least 8% by weight, and 0.1 to 5.0 wt.% of a noble metal such as Pt, Pd or the like is deposited on the pillared mica.

4. The process of any one of claims 1 to 3 wherein the specific surface area is between 200 m²/g and 450 m²/g.

5. The process of any one of claims 1 to 4 wherein the interlayer spacing of the mica is between 16 Å and 20 Å (16 x 10⁻¹⁰ to 20 X 10⁻¹⁰m).

6. The process of any one of claims 1 to 6 wherein the interlayer spacing is between 17.5 Å and 19.0 Å (17.5 X 10⁻¹⁰ to 19.0 x 10⁻¹⁰m).

7. A pillared fluorotetrasilicic mica having a specific surface area greater than 75 m²/gm, wherein the mica is pillared by alumina or zirconia or chromia and has an aluminum or zirconium or chromium content of at least 8% by weight.

8. A process for pillaring fluorotetrasilicic mica comprising the steps of:
  (a) producing a slurry of mica in a polar solvent;
  (b) adding an aqueous solution to an alumina or zirconia or chromia oligimer to the mica slurry, said oligimer being present in an amount greater than 3.0 meg $Al_{13}^{7+}$ per gram of mica;
  (c) adjusting the pH of the oligimer and mica slurry mixture to the acidic range;
  (d) maintaining the mixture for a period of time sufficient to produce an expanded mica; and
  (e) drying and calcining the expanded mica to produce a pillared mica.

THERMAL STABILITY OF PILLARED TSM

FIG.1

STEAM STABILITY OF PILLARED TSM

FIG.2

STEAM STABILITY OF PILLARED, WASHED TSM

INTENSITY

$518^{\circ}$C 298M$^2$/g

$617^{\circ}$C, 290M$^2$/g

$721^{\circ}$C, 279 M$^2$/g

0.00    5.00    10.00    15.00    20.00    25.00    30.00
2θ (DEGREES)

FIG.3

EP 0 341 023 A2

STEAM STABILITY OF PILLARED, WASHED TAENIOLITE

$518^{\circ}C$, $276 M^2/g$

$617^{\circ}C$, $188 M^2/g$

$721^{\circ}C$, $123 M^2/g$

INTENSITY

0.00     5.00     10.00     15.00     20.00     25.00     30.00

2θ (DEGREES)

FIG.4

EP 0 341 023 A2

FIG.

EP 0 341 023 A2